(19) Europäisches Patentamt / European Patent Office / Office européen des brevets

(11) **EP 3 664 784 B1**

(12) **EUROPEAN PATENT SPECIFICATION**

(45) Date of publication and mention
of the grant of the patent:
**06.04.2022 Bulletin 2022/14**

(21) Application number: **18769418.7**

(22) Date of filing: **10.08.2018**

(51) International Patent Classification (IPC):
**A61K 9/51** *(2006.01)*     **A61K 9/50** *(2006.01)*
**A61K 9/00** *(2006.01)*     **A61K 41/00** *(2020.01)*
**A61K 49/18** *(2006.01)*     **A61P 35/00** *(2006.01)*

(52) Cooperative Patent Classification (CPC):
**A61K 9/5192; A61K 9/0019; A61K 9/5115;**
**A61K 41/0052; A61K 49/183; A61P 35/00;**
A61K 9/5094

(86) International application number:
**PCT/GB2018/052282**

(87) International publication number:
**WO 2019/030535 (14.02.2019 Gazette 2019/07)**

(54) **MAGNETIC STRUCTURES**

**MAGNETISCHE STRUKTUREN**

**STRUCTURES MAGNÉTIQUES**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **10.08.2017 GB 201712879**

(43) Date of publication of application:
**17.06.2020 Bulletin 2020/25**

(73) Proprietor: **Hipermag Limited**
**Stroud, Gloucestershire GL6 9AN (GB)**

(72) Inventors:
• **BINNS, Christopher Robin**
**Stroud**
**Gloucestershire GL6 9AN (GB)**
• **BINNS, Robert Davidson**
**Stroud**
**Gloucestershire GL6 9AN (GB)**
• **KINMONT, Patrick William John**
**Stroud**
**Gloucestershire GL6 9AN (GB)**

(74) Representative: **Forresters IP LLP**
**Skygarden**
**Erika-Mann-Straße 11**
**80636 München (DE)**

(56) References cited:
GB-A- 2 543 604     US-A1- 2008 057 001
US-A1- 2012 168 669     US-A1- 2016 271 274

## Description

Field of the Invention

The present invention relates to a contrast agent for magnetic resonance imaging and its production process. Also disclosed, but not claimed, is an apparatus and a method for simultaneously or sequentially identifying and treating an illness, condition or disease of a patient. In particular, disclosed is an apparatus and a method for combining MRI and hyperthermia to simultaneously or sequentially diagnose and treat a disease of a patient.

Background to the Invention

Magnetic resonance imaging (MRI) is a medical imaging technique used in the diagnosis of a patient suffering from pain or discomfort. MRI is used in radiology to generate images of the anatomy and the physiological processes of the body of a patient. MRI has become one of the most widely used tools for non-invasive clinical diagnosis owing to its high degree of soft tissue contrast, depth of penetration and spatial resolution.

The human body is largely composed of water molecules, each containing two hydrogen nuclei, or protons. When inside the magnetic field (Bo) of the scanner, the magnetic moments of these protons align with the direction of the field.

A radio frequency pulse is then applied, causing the protons to alter their magnetization alignment relative to the field. In response to the force bringing them back to their equilibrium orientation, the protons undergo a rotating motion (precession). These changes in magnetization alignment cause a changing magnetic flux, which yields a changing voltage in receiver coils to give the signal. The frequency at which a proton or group of protons in a voxel resonates depends on the strength of the local magnetic field around the proton or group of protons. By applying additional magnetic fields (gradients) that vary linearly over space, specific slices or portions of a body to be imaged can be selected, and an image is obtained by taking the 2-D Fourier transform of the spatial frequencies of the signal (a.k.a., k-space). Diseased tissue, such as tumours, can be detected because the protons in different tissues return to their equilibrium state at different rates (i.e., they have different relaxation times). By changing the parameters on the MRI apparatus this effect is used to create contrast between different types of body tissue.

MRI may involve administering a contrast agent having magnetic properties to a patient prior to scanning. The contrast agent may be injected into and circulate through the bloodstream and may be absorbed by certain tissues of a patient. The contrast agents typically comprise inorganic nanoparticles such as for example iron oxide nanoparticles or ferrites. On application of strong magnetic fields, the contrast agent in a similar way to the protons of water molecules emits signals which are visible on the generated image. The location of the contrast agents may help the medical practitioner to diagnose an illness or disease of the patient.

MRI is used to image every part of the body, and is particularly useful for neurological conditions, for disorders of the muscles and joints, for evaluating tumours, and for showing abnormalities in the heart and blood vessels.

Conventionally, a patient is first assessed using magnetic resonance imaging (MRI) to diagnose an illness, condition or disease. Once the diagnosis has been made, the patient is then separately treated. As a result, the process for diagnosing and treating a patient involves multiple hospital appointments and can require a significant amount of a medical practitioner's time.

The present invention may advantageously provide an improved apparatus for and a method of diagnosing and treating a patient which has improved efficiency compared to conventional processes and significantly reduces the time required. The present invention may advantageously further provide an improved apparatus for and a method of simultaneously or sequentially diagnosing and treating a patient. The present invention may also advantageously further provide an improved apparatus for and a method of simultaneously or sequentially diagnosing and treating a patient using magnetic resonance of a contrast agent comprising nanoparticles. The present invention may also advantageously further provide an improved contrast agent comprising nanoparticles for use in magnetic resonance imaging.

US2008057001 A1 discloses contrast agents and methods for making them that use Fe nanoparticles that produce higher clarity and particularity in MRI imaging. Various alloys and core compounds are disclosed that may be used to produce higher clarity MRI images.

US2012168669 A1 discloses a composite nanoparticle, for example a nanoparticle containing one or a plurality of cores embedded in another material. A composite nanoparticle can be formed by a one step process that includes: ejecting material from a bulk target material using physical energy source, with the bulk target material disposed in a liquid. Composite nanoparticles are formed by cooling at least a portion of the ejected material in the liquid. The composite fine particles may then be collected from the liquid. A product that includes composite fine particles may be formed with laser ablation, and ultrashort laser ablation may be utilized so as to preserve composite nanoparticle stoichiometry. For applications of the composite fine particles, optical properties and/or magnetic properties may be exploited for various applications.

US2016271274 A1 discloses magnetic iron oxide nanoparticles (MIONs) having silica (SiMION) and gold-silica (AuSiMION) nanoshells, methods of their preparation, and their use in cancer imaging and therapy applications.

GB2543604 A discloses a liquid dispersible powder comprising nanoscale grains of a matrix material containing one or more isolated nanoparticles. A composition for the magnetic nanoparticle hyperthermia (MNH) treatment of tumours comprising nanoscale grains of a matrix material containing one or more isolated nanoparticles is also disclosed. The nanoparticles are preferably around 15nm in size. Preferably at least a portion of the nanoparticles comprise magnetic nanoparticles of a magnetic core selected from one or more of iron or an alloy of iron and cobalt encased in a biocompatible shell which may be an FeO shell. A method of production of a liquid-dispersible powder comprising the steps of: (a) providing nanoparticles prepared under ultra-high vacuum (UHV) gas phase conditions; (b) co-depositing the nanoparticles within a matrix material under UHV gas phase conditions; and (c) grinding the film to a fine powder comprising grains of groups of matrix isolated nanoparticles, is also disclosed. Preferably the grinding step comprises ball milling.

Statement of Invention

According to a first aspect of the present invention there is provided a process according to claim 9 of producing a contrast agent for use in magnetic resonance imaging, the process comprising:
forming magnetic nanoparticles by depositing at least in part a layer of metal on a core of ferromagnetic material, in which the core and the layer of metal are comprised or composed of different materials.

Magnetic materials find widespread use in modern technology and are to be found in nearly all electromechanical apparatus. The performance of magnetic materials in respect of their secondary parameters, such as coercivity and energy product, has improved greatly over the last century. There has nevertheless been little improvement in the most fundamental property, i.e. saturation magnetisation, which determines the strength of produced magnetic field. The most magnetic material for use in electro-mechanical apparatus, i.e., $Fe_{60}CO_{40}$ alloy, has been available since the 1920s and until recently there has been no material found with a higher magnetisation.

The most direct measure of saturation magnetisation is the magnetic moment per atom which is specified in Bohr magnetons ($\mu_B$). The magnetic moment for pure Fe is 2.22 $\mu_B$ per atom whereas for $Fe_{60}CO_{40}$ alloy the magnetic moment is 2.45 $\mu_B$ per atom. The latter value, i.e., 2.45 $\mu_B$ per atom, is termed the Slater-Pauling limit and was believed to be the ultimate magnetisation available from transition metal alloys. Generally, the efficiency of electro-mechanical apparatus improves as the square of the magnetisation of the magnetic material. Even small increases in magnetisation are therefore valuable especially in green technologies such as electric vehicles and wind turbines.

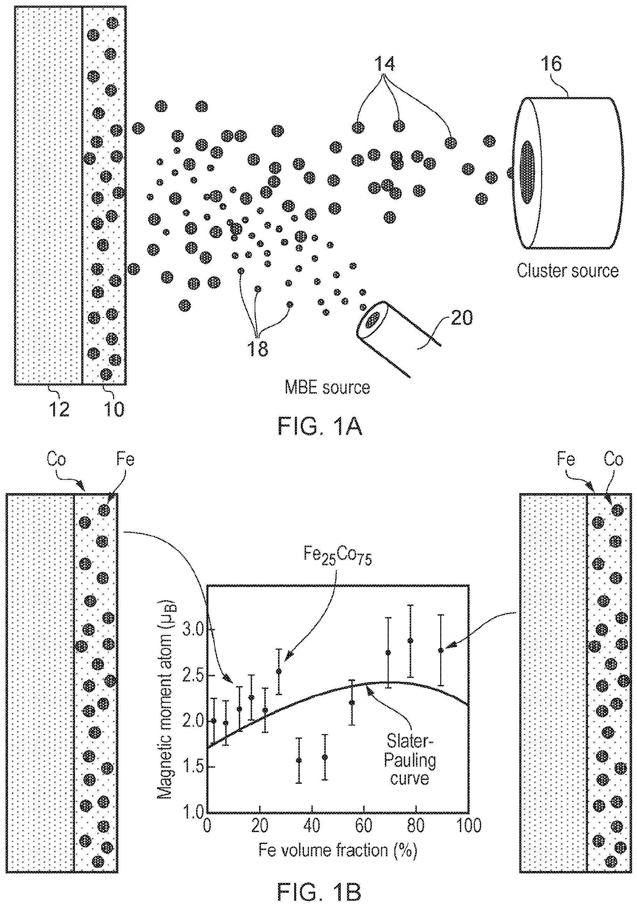

Upon development in the early 1990s of gasphase nanoparticle sources capable of depositing nanoparticles with diameters in the range of 1 to 5 nm it was discovered that the magnetic moments per atom of Fe, Co and Ni nanoparticles with diameters no more than about 5 nm are significantly higher than for bulk structures formed from the same material. In view of this, magnetic structures in which nanoparticles of one of Fe and Co are embedded in a matrix of the other of Fe and Co have been developed with such magnetic structures having a magnetisation which exceeds the magnetisation of $Fe_{60}CO_{40}$ alloy to thereby break the Slater-Pauling limit for the first time. Figure 1A illustrates the formation of one such magnetic structure. As shown in Figure 1A a magnetic structure 10 is formed by co-deposition on a substrate 12 of Fe nanoparticles 14 from a cluster source 16 and of Co matrix material 18 from a Molecular Beam Epitaxy (MBE) source 20. Co-deposition of Fe nanoparticles and Co matrix material results in a structure in which Fe nanoparticles are distributed through and embedded in the Co matrix. According to an alternative approach a magnetic structure in which Co nanoparticles are distributed through and embedded in an Fe matrix is formed by co-deposition of Co nanoparticles from the cluster source and of Fe matrix material from the MBE source.

The magnetic moment per atom for each of a structure having Fe nanoparticles in a Co matrix and a structure having Co nanoparticles in an Fe matrix are shown in Figure 1B as a function of the Fe volume fraction. Figure 1B also shows the Slater-Pauling curve for $Fe_{60}CO_{40}$ alloy as a function of the Fe volume fraction. As can be seen from Figure 1B the best results are obtained from Co nanoparticles embedded in an Fe matrix which yields values approaching 3 $\mu_B$ per atom. At lower Fe volume fractions the magnetic moment per atom for Fe nanoparticles embedded in a Co matrix exceeds the corresponding value defined by the Slater-Pauling curve. The improvement is seen because the fundamental building blocks of the material already have an enhanced magnetisation and also because the matrix itself has a nanostructure which leads to enhanced moments. More specifically there is a higher proportion of atoms at a surface or interface in a nanostructure (approaching 50% in the presently described structure) with each such atom having enhanced spin and orbital moments. On the other hand, and as can be seen from the left half of the graph of Figure 1B the magnetisation falls below the Slater-Pauling curve at Fe volume fractions of more than about 20% which is the percolation threshold.

The present inventors have appreciated that the high level of aggregation of Fe nanoparticles at higher levels of Fe volume fractions produces a phase separated mixture of macroscopic grains and the magnetisation falls as a consequence to a weighted average of the magnetic moments of Co (1.7 $\mu_B$ per atom) and Fe (2.22 $\mu_B$ per atom). This is the reason for the fall in the magnetisation to below the Slater-Pauling curve as seen in Figure 1B. As disclosed in International Patent Application no. PCT/GB2013/052426, the present inventors have further appreciated that an improvement in performance may be gained by providing for an increase in the nanoparticle volume fraction without marked aggregation. The invention has been devised in the light of the above-mentioned appreciation.

According to a another aspect of the present invention there is provided a process according to claim 9 of producing a contrast agent for use in magnetic resonance imaging, the process comprising:

depositing a matrix material onto a substrate to thereby form a matrix; and
depositing magnetic nanoparticles onto the matrix as the matrix forms to thereby embed the magnetic particles in the matrix, in which each magnetic nanoparticle comprises a core covered at least in part with a layer of metal, and in which at least one of the matrix material and the core is of ferromagnetic material, and the core and the layer of metal are of different materials.

In use, the source of matrix material and the source of magnetic particles may be operated simultaneously to thereby deposit the matrix material and the magnetic particles simultaneously. The source of matrix material and the source of magnetic particles may be configured to direct a beam of matrix material and a beam of magnetic particles towards the substrate. A matrix in which magnetic particles are embedded is thereby formed. The source of magnetic particles may comprise a gas phase source and more specifically a cluster beam source, such as a gas aggregation source. The source of matrix material may comprise an evaporator, for example, a thermal evaporator such as an MBE source or sputtering apparatus. The apparatus may further comprise an evaporator such as a thermal evaporator which is operable to deposit the layer of metal on the core of each magnetic particle. The evaporator may be disposed between a source of particle cores and the substrate. In addition, the evaporator may be configured to define a space through which a beam of particle cores pass, the evaporator being operative to form a vapour of metal in the space whereby the vapour impinges upon a surface of each particle core. The apparatus may further comprise an arrangement, such as a venturi, operable to accelerate a beam of magnetic particles before their deposition onto the matrix. The apparatus may further comprise a temperature reducing arrangement, such as refrigeration apparatus, which is operable to reduce a temperature of the matrix as it forms on the substrate.

In use the matrix material and the magnetic particles are deposited to form a matrix on the substrate in which the magnetic particles are embedded. The matrix material and the magnetic particles may be deposited simultaneously, for example, by simultaneous operation of a source of matrix material, such as a Molecular Beam Epitaxy (MBE) source, and a source of magnetic particles, such as a thermal gas aggregation source. A magnetic structure may thereby be formed in which magnetic particles are distributed through and embedded in the matrix. The magnetic structure is typically formed as a thin film on the substrate. Covering the core at least in part with a layer of metal of different material composition to the core reduces the likelihood of cores coming into contact at particle volume fractions higher than the percolation threshold. Aggregation of magnetic particles is therefore reduced and an increase in magnetisation seen over structures formed from magnetic particles lacking the layer of metal.

The process is suited to the deposition of nanoparticles onto the matrix. The step of depositing magnetic particles may therefore comprise depositing magnetic nanoparticles. Each magnetic particle may be of a diameter of no more than substantially 100 nm, 60 nm, 30 nm, 20 nm, 15 nm or 10 nm. The magnetic moment per atom of magnetic nanoparticles of diameter between substantially 1 nm and substantially 5 nm has been found to be significantly higher than for bulk structures formed from the same material. Each magnetic particle may therefore be of a diameter of no more than substantially 5 nm, 4 nm, 3 nm, 2 nm or 1 nm. Each magnetic particle may be of a diameter of more than substantially 0.5 nm, 1 nm, 2 nm, 3 nm or 4 nm. Magnetic particles having a diameter less than 2 nm may advantageously be excreted by the kidneys. The layer of metal may have a thickness of no more than substantially 4 nm, 2 nm, 1 nm, 0.8 nm, 0.6, 0.4 nm or 0.2 nm. Considering the thickness of the metal layer in terms of atomic layers, the thickness may be between 1 and 10 atomic layers such as no more than 10, 8, 6, 4, 2 or 1 atomic layers. For example, where a core has a diameter of substantially 5 nm the layer of metal may be a single atomic layer thick such as substantially 0.2 nm thick. The layer of metal may substantially cover the core. A surface of the layer of metal may define an exterior surface of the magnetic particle. The matrix material may be of the same material as the layer of metal. For example, each magnetic particle may comprise a Fe core covered at least in part with a layer of Co and the matrix material may be Co. By way of further example each magnetic particle may comprise a Co core covered at least in part with a layer of Au and the matrix material may be Au. Using the same material for the layer and the matrix may be advantageous in hipermag (high performance magnetic structures) applications. Use of the same material may reduce the likelihood of the particle cores coming into contact even at volume fractions much higher than the percolation threshold.

The core comprises a ferromagnetic material, such as a ferromagnetic element. More specifically the ferromagnetic material is a ferromagnetic transition metal, such as one of Fe, Co and Ni. The matrix material may be a metal and more specifically one of a transition metal and a rare earth metal. The core and the matrix material may be formed from different materials. Where the matrix material is a transition metal the transition metal may be ferromagnetic or diamagnetic. The metal layer may be one of a transition metal and a rare earth metal.

The metal layer may be a ferromagnetic or diamagnetic transition metal. For hipermag applications it is preferred that each magnetic particle comprises a core formed from a ferromagnetic transition metal and either a ferromagnetic transition metal layer or a diamagnetic transition metal layer. The diamagnetic transition metal may be a Group 11 metal such as gold or silver. Thus the core/layer composition for hipermag applications may be Fe/Co, Co/Fe, Fe/Ag, Co/Ag, Fe/Au or Co/Au. As mentioned above use of the same material for the layer and the matrix may be advantageous in hipermag applications such that the matrix is formed from a metal layer matching ferromagnetic or diamagnetic transition metal. Deposition of the magnetic particles may be by way of vacuum assisted deposition of magnetic particles in the gas phase and more specifically by way of deposition of a beam of gas-phase magnetic particles. The process may thus comprise causing a beam of magnetic particles to impinge upon the matrix as the matrix forms. The beam may be generated by a gas phase source and more specifically by a cluster beam source, such as a gas aggregation source. The gas phase source may be operative to produce a beam of particle cores absent their layer of metal. A layer of metal may be provided on each core as described herein below. The different forms of gas-phase source generate particles in different size ranges when operated at optimum flux (output) levels. A sputter gas aggregation source normally generates particles having a diameter of about 10 nm when operative at an optimum flux level. A thermal gas aggregation source normally generates smaller particles of about 2 nm diameter when operative at an optimum flux level. The thermal gas aggregation source is often preferred for the formation of high performance magnetic structures (so called hipermags). Deposition of the matrix material may be by way of vacuum assisted deposition of the matrix material in the gas phase and more specifically by way of deposition of an atomic beam of matrix material. The process may thus comprise causing an atomic beam of matrix material to impinge upon the substrate. The atomic beam may be generated by an evaporator, for example, a thermal evaporator such as an MBE source or by sputtering. Forming the magnetic structure by means of an atomic beam of matrix material from an evaporator and a beam of magnetic particles from a cluster source may confer the advantage of providing for independent control over the grain size and volume fraction in the magnetic structure.

The process comprises depositing the layer of metal on the core of each magnetic particle. Deposition of the layer of metal may be by vacuum assisted deposition of metal vapour. Metal vapour may therefore be provided in a same vacuum as a source of particle cores. The metal vapour may be generated by an evaporator such as a thermal evaporator. The temperature of the thermal evaporator may be determined by the metal to be deposited, e.g. 800 °C for silver and 1000 °C for iron. A thickness of the layer of metal may be controlled by varying the operative temperature of the thermal evaporator in view of it normally being impossible to change the velocity of the particle core beam. The evaporator may be disposed between the source of particle cores and the substrate. In addition, the evaporator may be configured to define a space through which a beam of particle cores pass, the evaporator being operative to form a vapour of metal in the space whereby the vapour impinges upon a surface of each particle core. The evaporator may be configured to surround the beam of particle cores. The evaporator may therefore provide for improved coverage of the whole surface of the particle cores. The evaporator may, for example, define a tube through which the beam of particle cores passes. The process may further comprise accelerating a beam of magnetic particles before their deposition onto the matrix. Acceleration may be after deposition of a layer of metal. Acceleration may be achieved by means of apparatus which is configured to interact with the beam of magnetic particles. A venturi may, for example, be used.

Each magnetic particle may comprise a plurality of layers over the core. The layers may be formed from the same material as each other or one another or different material to each other or one another. The process may therefore comprise a deposition step for each layer. Deposition of each layer may be by vacuum assisted deposition from a thermal evaporator as described above. The plural evaporators may be disposed in line whereby, for example, a first evaporator provides for deposition of a first layer and a second evaporator provides for deposition of a second layer.

The heavy rare earth metals have much higher magnetic moments than transition metals. For example, the magnetic moment of Dy reaches 10 $\mu$B per atom. Generally rare earth metals are of themselves of limited use for technology on account of their low Curie temperature, which for most rare earth metals is below room temperature, and their magnetic hardness, which requires very high magnetic fields to achieve saturation. Incorporation of transition metals raises the Curie temperature and makes the rare earth and transition metal composition magnetically softer. On the other hand, the transition metal magnetically polarises in the opposite direction to the rare earth metal whereby the moment of the composition rapidly decreases as the volume fraction of the transition metal is increased. This problem may be addressed by providing magnetic particles having the structure described above. Each magnetic particle may therefore comprise a core formed from a transition metal covered at least in part with a layer of an antiferromagnetic material and more specifically an antiferromagnetic transition metal, such as Cr or Mn. The matrix material comprises a rare earth metal, such as Dy or Ho. The core comprises a ferromagnetic transition metal, such as Fe or Co. The layer of antiferromagnetic material may induce a parallel alignment of the magnetic moment of the rare earth matrix and the transition metal core. The thickness of the layer of antiferromagnetic material may be between 1 and 10 atomic layers such as no more than 10, 8, 6, 4, 2 or 1 atomic layers. In addition, each magnetic particle may comprise a second layer which covers the layer of antiferromagnetic material at least in part, the second layer being formed from a rare earth metal. More specifically the second layer and the matrix may be of the same rare earth metal. The thickness of the second layer may be between 1 and 10 atomic layers such as no more than 10, 8, 6, 4, 2 or 1 atomic layers. Provision of the second layer may decrease agglomeration of magnetic particles in the magnetic structure.

The substrate on which the magnetic structure is formed may constitute a component forming part or to form part of a product. For example, the magnetic structure may be formed on part of a roll of material in reel to reel coating apparatus. By way of further examples, the substrate may be a component forming part or to form part of the like of an electric motor or mobile telephone. As mentioned above the magnetic structure is typically formed as a thin film on the substrate. Formation of the magnetic structure as a bulk structure is normally much less readily achieved. Nevertheless, the magnetic structure may be formed on a critical magnetic component, e.g. at strategic locations, in the like of electro-mechanical apparatus to amplify the magnetic field of the magnetic component. The present inventors have appreciated the formation of a magnetic structure comprising magnetic particles having a core and a layer of metal to be of wider application than hitherto described. For example, an advantage of the use of Au or Ag as either a first or subsequent layer over the core of the magnetic particles is that such magnetic particles can be deposited in a liquid nitrogen cooled matrix of ice. When the matrix with its embedded magnetic particles is complete the temperature is allowed to increase and the resulting magnetic particle containing liquid can be sprayed onto a desired surface to thereby deposit the magnetic particles upon the surface. The process may therefore comprise depositing a matrix material on a substrate to form a solid matrix, depositing magnetic particles comprising a layer of diamagnetic transition metal onto the matrix as the matrix forms and raising the temperature of the thus formed magnetic structure to thereby form a liquid containing magnetic particles. The diamagnetic transition metal may be a Group 1 1 metal, such as gold or silver. The process may comprise depositing a matrix material of liquid form at ambient, e.g. room, temperature onto a substrate in an environment such as to form a solid matrix. An environment such as to form a solid matrix may be created in dependence on control of at least one of temperature and pressure. The environment may be created by means of a temperature reducing arrangement, such as refrigeration apparatus. The matrix material may comprise a compound and more specifically a polar molecular compound such as water.

According to a further aspect of the present invention, there is provided a contrast agent according to claim 1 for magnetic resonance imaging, the contrast agent comprising a plurality of magnetic nanoparticles, in which each magnetic nanoparticle comprises a core comprising or composed of ferromagnetic material covered at least in part with a layer of metal, and in which the core and the layer of metal are comprised or composed of different materials.

According to a aspect of the present invention, there is provided a contrast agent according to claim 1 for magnetic resonance imaging, in which the contrast agent comprises:

> a plurality of magnetic nanoparticles, in which each magnetic nanoparticle comprises a core covered at least in part with a layer of metal; and
> a matrix,
> in which the magnetic nanoparticles are embedded in the matrix, and in which at least one of the matrix and the core is of ferromagnetic material, and in which the core and the layer of metal are comprised or composed of different materials.

According to a further aspect, which is not claimed, the disclosure provides a contrast agent in the form of a liquid-dispersible powder comprising nanoscale grains of a matrix material containing a plurality of isolated nanoparticles, wherein at least a portion of the nanoparticles comprise magnetic nanoparticles, wherein each magnetic nanoparticle comprises a magnetic core selected from one of more of Fe or an alloy of Fe/Co encased in a biocompatible FeO shell. The term "nanoparticles" used herein is intended to include simple elemental, core/shell, and alloy structures.

A further aspect, which is not claimed, provides a method of production of a contrast agent in the form of a liquid-dispersible powder as described herein, the method comprising the steps of:

> a. providing nanoparticles prepared under ultra-high vacuum (UHV) gas phase conditions;
> b. co-depositing the nanoparticles within a matrix under UHV gas phase conditions;
> c. grinding the film to a fine powder comprising grains of groups of matrix isolated nanoparticles.

The grinding step may comprise ball milling, however, any suitable method may be used to grind the film provided it results in a suitably fine powder. Such powder has to be sufficiently fine to pass through the smallest capillary of the body.

Typically, the nanoparticles are around 15 nm in diameter. As a nanoscale grain of matrix material may be as large as 100 nm in width, it can therefore be appreciated that each grain of matrix material may retain several embedded nanoparticles therein thereby allowing the dispersal of nanoparticles, and particularly magnetic nanoparticles, within liquid such as but not limited to water, whilst minimising agglomeration.

Dispersal of magnetic nanoparticles in liquid leads to agglomeration with consequential altering of the desirable properties of the particles. By embedding the nanoparticles within a matrix material, the nanoparticles are inherently spatially separated from one another and agglomeration is minimised. The nanoscale nature of the grains of matrix material are sufficiently big to contain one or more nanoparticles per grain whilst remaining small enough to be liquid-dispersible, for example water-dispersible, and therefore useful in medical applications.

The contrast agent is preferably provided in the form of a liquid-dispersible powder, for example a water-dispersible powder. Alternatively, the contrast agent may be provided in the form of a liquid-based solution, for example water-based solution, in which the grains of matrix have already been dispersed.

A further aspect, which is not claimed, is a method of reducing the agglomeration of nanoparticles of the contrast agent in the form of a liquid dispersible powder in water, the method comprising isolating nanoparticles in nanoscale grains of a matrix material. According to a further aspect of the present invention there is provided a pharmaceutical composition comprising a contrast agent as herein described. The pharmaceutical composition may further comprise at least one pharmaceutically acceptable carrier.

As used herein "pharmaceutically acceptable carrier" means a carrier that is useful in preparing a pharmaceutical composition or formulation that is generally safe, non-toxic, and neither biologically nor otherwise undesirable, and may include conventional liquid carriers such as water, saline, ethanol, polyols (such as glycerol, propylene glycol, polyethylene glycol, and the like), vegetable oils, such as olive oil, and injectable organic esters, such as ethyl oleate, and suitable mixtures thereof.

The carrier should be suitable for intravenous, intramuscular, subcutaneous, parenteral, spinal or epidermal administration (e.g., by injection or infusion). The carrier is advantageously sterile at the time the contrast agent is administered.

The pharmaceutical composition may be for use in therapy, for the diagnosis and treatment of an illness, condition or disease of a patient.

A further aspect of the present invention provides a contrast agent or a pharmaceutical composition as described herein for use in the diagnosis and treatment of an illness, condition or disease of a patient. The illness, condition or disease may for example be selected from one or more of: tumours (i.e. caused by cancer), viruses, bacterial infections, inflammatory conditions, or any combination thereof. In one embodiment, the contrast agent or pharmaceutical composition may be used for the diagnosis and treatment of magnetic nanoparticle hyperthermia (MNH) treatment of tumours.

The contrast agent or pharmaceutical composition may be administered to the patient by any suitable means. For example, the contrast agent or pharmaceutical composition may be topically administered, for example sprayed, ingested, intravenously administered, or any combination thereof.

The contrast agent of the present invention may concentrate themselves adjacent damaged tissue due to one or more of: enhanced permeability effect (EPR) of tumours; direct injection into the desired tissue; and/or by the application of an external magnetic field, for example an external static magnetic field.

MNH is an emerging cancer treatment, based on the fact that magnetic nanoparticles produce heat when exposed to an alternating magnetic field. The nanoparticles of the contrast agent or pharmaceutical composition of the present invention may be configured to target tumours either by the body's natural processes such as EPR, magnetic targeting or biological targeting. then exposing the cells to an alternating magnetic field of predetermined amplitude and frequency, the cell temperature would rise thereby encouraging apoptosis of the cells without harm to surrounding tissues.

The nanoparticles may be configured to specifically target certain biological targets or receptors. The nanoparticles may comprise a further coating of biological target specific ligands, antibodies, stem cells or any combination thereof. The nanoparticles may for example be coated with a layer of liposomes, antibodies, stem cells or any combination thereof. The liposomes, antibodies, or stem cells may be surface modified in order to endow them with multiple functionalities including increased accumulation at the target tissue, for example tumour tissue and organelle specific drug delivery.

It is known that due to the nature of the tumour-associated blood vessels, biomacromolecules and nanosized drug delivery systems readily translocate across the capillary endothelium and enter the interstitial space. The size of the gaps between the endothelial cells lining the tumour capillaries ranges from 100 to 780 nm depending on the type of tumour. A typical normal endothelium has gaps of between 5 and 10 nm. It is also known that tumours tend to lack adequate lymphatic drainage. As a result, it has been found there is an accumulation of macromolecules and nanoparticles in the tumour microenvironment due to enhanced permeability retention of a tumour. The contrast agent of the present invention may therefore be used to target tumours within a patient by relying solely on the pathophyiosological properties of the tumour tissues. This is referred to as passive drug targeting. The nanoparticles may comprise a layer of liposomes to improve the uptake across the capillary endothelium.

The nanoparticles may comprise a layer of actively targeted liposomes which have been specifically modified or selected to minimise off target effects and to optimise affinity for target body tissue. The layer of actively targeted liposomes may comprise for example, conjugated moieties including small-molecule ligands, peptides and monoclonal antibodies, on the surface of the liposome layer. Liposomes that accumulate in the tumour microenvironment can be subsequently endocytosed into the cells by interacting with specific cell surface receptors. To efficiently target the contrast agent of the present invention to cancer cells, the liposome layer may comprise specific targeting moieties in sufficient quantities to provide have optimum affinity for the cell surface receptors of the tumour or target body tissue.

The layer of liposome may for example be a folate conjugated liposome layer. It is known that folate receptors are greatly overexpressed by many tumour cell types. Furthermore, overexpression of the folate receptors on macrophages is an indication of inflammatory diseases such as psoriasis, Chron's disease, rheumatoid arthritis and atherosclerosis. As such, the contrast agent of the present invention may also be used to diagnose and treat inflammatory diseases.

According to a further aspect, which is not claimed, there is provided a magnetic resonance imaging apparatus for simultaneously or sequentially diagnosing and treating an illness or condition of a patient to whom the contrast agent or pharmaceutical composition of previous aspects of the invention has been administered, the apparatus comprising:

a source of a radio frequency pulse operable to expose a patient located within the apparatus to a radio frequency pulse; and
a controller operable to vary the field amplitude of the radio frequency pulse.

The controller is preferably operable to vary the field amplitude between at least a first amplitude and a second amplitude. The controller may be operable to vary the field amplitude between a plurality of amplitudes. The controller may be operable to vary the field amplitude between a plurality of discrete amplitudes. The controller may be operable to vary the field amplitude between a continuous range of amplitudes.

The term "sequentially" is used herein to refer to the apparatus being used in a single one step process to provide both diagnosis and treatment and in which the diagnosis occurs prior to the treatment.

The apparatus may further comprise a contrast agent source comprising a contrast agent or pharmaceutical composition as herein described.

According to a further aspect, which is not claimed, there is provided a method of simultaneously or sequentially diagnosing and treating an illness, disease or condition of a patient, the method comprising:

administering a contrast agent or pharmaceutical composition as herein described to a patient;
positioning the patient within an apparatus as herein described;
operating the controller such that the source of a radio frequency pulse is provided at a first field amplitude and first field frequency sufficient to generate one or more images of one or more parts of the body of the patient;
identifying an illness, disease or condition associated with the patient from the generated one or more images; and
operating the controller such that the source of a radio frequency pulse is provided at a second field amplitude and second field frequency sufficient to treat the illness, disease or condition associated with the patient.

Also disclosed, but not claimed, is a method of diagnosing a disease or condition of a patient, to whom the contrast agent or pharmaceutical composition of previous aspects of the invention has been administered, comprising the steps of:

positioning the patient within an apparatus as herein described;
operating the controller such that the source of a radio frequency pulse is provided at a first field amplitude and first field frequency sufficient to generate one or more images of one or more parts of the body of the patient;
identifying an illness, disease or condition associated with the patient from the generated one or more images.

The method may also comprise the step of operating the controller such that the source of a radio frequency pulse is provided at a second field amplitude and second field frequency sufficient to treat the illness, disease or condition associated with the patient.

Preferably, the identification of the illness, disease or condition of the patient occurs prior to the subsequent treatment of the illness or condition.

The first field amplitude is preferably a low field amplitude. Preferably the first field frequency is a low field frequency. The first field amplitude may be a minimum low field amplitude. The first field frequency may be a minimum low field frequency. Preferably, the first field frequency is 25 kHz. Preferably the first field amplitude is 20 mT/$\mu_o$. The first field amplitude and first field frequency are selected for providing one or more images of one or more parts of the body of the patient.

The second field amplitude is preferably a high field amplitude. Preferably the second field frequency is a high field frequency. The second field amplitude may be a maximum low field amplitude. The second field frequency may be a maximum low field frequency. Preferably, the second field frequency is 100 kHz. Preferably the second field amplitude is in the range of between 20 mT/$\mu_o$ and 40 T/$\mu_o$. The second field amplitude is preferably higher than the first field amplitude. The second field amplitude and second field frequency are selected for treating the illness, disease or condition associated with the patient.

The second field amplitude and/or the second field frequency are preferably sufficient to raise the temperature, by for example a couple of degrees, in the region of and adj acent to the contrast agent of the present invention compared to the temperature of the rest of the body. For example, the second field amplitude and/or frequency may be sufficient to generate a localised hot spot to raise the temperature of the desired tissue. By raising the temperature of the tissue in the region or adjacent the contrast agent, which has been localised at the site of the illness, condition or disease, the contrast agent or pharmaceutical composition of the present invention preferably encourage apoptosis of the adjacent tissue cells, for example cancer cells, without harming surrounding healthy tissue cells.

The source of radio frequency pulse is preferably applied to the tissue of a patient to produce a localised hot spot which is applied as a raster effect. In particular, the source of radio frequency pulse is applied to run back and forth across the desired portion of affected tissue as identified during the imaging stage of the method so as to generate a heat spot which is directed to run back and forth across the desired portion of affected tissue. By applying the pulse to generate a hot spot to produce a raster effect, the method of the present invention provides a more selective and directed approach to treatment of the illness, condition or disease and thereby minimises damage to any nearby adjacent tissue.

The steps of diagnosis and treatment are preferably automated.

After the first stage of treatment of the identified affected tissue, the method may further comprise repeating the imaging phase by operating the controller to provide the source of a radio frequency pulse at a first field amplitude and first field frequency sufficient to generate a second series of one or more images of the one or more previously affected parts of the body of the patient.

The method may further comprise identifying whether the previously affected parts of the body are still affected from the generated second series of one or more images.

The method may further comprise, if necessary, operating the controller such that the source of a radio frequency pulse is provided at a second field amplitude and second field frequency sufficient to treat any remaining illness, disease or condition within the affected parts of the body.

The method may continue to comprise any suitable number of repeated imaging steps by operating the controller to provide the source of a radio frequency pulse at a first field amplitude and first field frequency sufficient to generate further series of one or more images of the one or more previously affected parts of the body of the patient, and any suitable number of repeated treatment steps by operating the controller such that the source of a radio frequency pulse is provided at a second field amplitude and second field frequency sufficient to treat any remaining illness, disease or condition within the affected parts of the body.

The imaging and treatment steps may be repeated until the affected tissue has been effectively treated.

The contrast agents and pharmaceutical compositions of the present invention have been found to produce an order of magnitude more heat per gram of material than commercially available FeO nanoparticles which do not generate sufficient heat for efficient tumour therapy.

The disclosure provides, but does not claim, an apparatus and a method for simultaneously or sequentially diagnosing and treating an illness or condition of a patient during a single operation of the apparatus. The disclosure provides, but does not claim, an apparatus and a method for accurately identifying, diagnosing and locating an illness or condition of a patient while simultaneously or sequentially accurately locating and treating the illness or condition of a patient. The disclosure provides, but does not claim, an apparatus and a method with improved selectivity for treating an illness or condition of a patient. The apparatus and method therefore enables an illness or condition of a patient to be identified and treated, either simultaneously or sequentially, during a single operation of the apparatus and as such reduces the number of hospital appointments required by the patient and reduces the attendance time of a medical practitioner. As a result of the improved selectivity of the apparatus and method for treating an illness or condition of a patient, the recovery time of a patient is significantly reduced.

Another aspect of the invention relates to a contrast agent according to claim 1 for magnetic resonance imaging, in which the contrast agent comprises: a plurality of magnetic nanoparticles, wherein each magnetic nanoparticle comprises a core covered at least in part with a layer of metal, wherein the core and the layer of metal are comprised or composed of different materials; and one or more pharmaceutically acceptable carriers.

The term metal as defined herein includes, but is not limited to, alkali metals, alkaline earth metals, transition metals, and alloys and/or oxides thereof.

According to the claims, the contrast agent comprises a matrix. The plurality of magnetic nanoparticles is embedded in the matrix. Suitably, the core comprises a ferromagnetic material. Suitably, the layer of metal may comprise a diamagnetic transition metal selected from Ag or Au. The matrix may comprise a transition metal and comprises a rare earth metal.

The transition metal may be a ferromagnetic transition metal selected from Fe, Co, Ni, or a diamagnetic transition metal selected from Ag or Au. The rare earth metal may be selected from Dy or Ho. In some embodiments, the matrix and the layer of metal may be comprised or composed of the same material.

According to the claims, the core comprises a transition metal, the layer of metal comprises an antiferromagnetic material, and the matrix material comprises a rare earth metal. Suitably, the layer of metal comprises an antiferromagnetic transition metal and the core comprises a ferromagnetic transition metal. The ferromagnetic transition metal may be selected from Fe, Co, or Ni. The antiferromagnetic transition metal may be selected from Cr or Mn. The rare earth metal is selected from Dy or Ho.

The contrast agent may comprise a second layer which covers the layer of metal at least in part, the second layer being formed from a rare earth metal. Optionally, the second layer and the matrix may be comprised or composed of the same rare earth metal.

In some embodiments, the core may comprise a ferromagnetic transition metal, and the layer of metal may comprise a diamagnetic transition metal. The diamagnetic transition metal may be selected from Ag or Au.

In an embodiment, each magnetic nanoparticle may be of a diameter of no more than one of 10 nm, 5 nm, 4 nm, 3 nm, 2 nm, or 1 nm. Each magnetic nanoparticle may be of a diameter between 1 nm and 5 nm. For example, the diameter may be about 4 nm, or the diameter may be about 2 nm. In some embodiments, each magnetic nanoparticle may be of a diameter of no more than 2 nm.

Another aspect of the invention relates to a process for producing a contrast agent for use in magnetic resonance imaging, the process comprising: forming magnetic nanoparticles by depositing at least in part a layer of metal on a core, in which the core and the layer of metal are comprised or composed of different materials; and combining with one or more pharmaceutically acceptable carriers. Suitably, the core comprises a ferromagnetic material.

The process also comprises:

depositing a matrix material onto a substrate to thereby form a matrix; and depositing the magnetic nanoparticles onto the matrix as the matrix forms to thereby embed the magnetic nanoparticles in the matrix. Suitably, the core and the layer of metal are comprised or composed of different materials. The process may comprise depositing magnetic particles comprising a layer of diamagnetic transition metal onto the matrix as the matrix forms and raising the temperature of the thus formed magnetic structure to thereby form a liquid containing magnetic particles and optionally wherein the diamagnetic transition metal is a Group 11 metal and the matrix material comprises a polar molecular compound. Suitable Group 11 metals include, for example, Ag and Au.

Another aspect which is not claimed relates to a contrast agent in the form of a liquid-dispersible powder comprising nanoscale grains of a matrix material containing a plurality of isolated nanoparticles, wherein at least a portion of the nanoparticles comprise magnetic nanoparticles, wherein each magnetic nanoparticle comprises a magnetic core selected from one of more of Fe or an alloy of Fe/Co encased in a biocompatible FeO shell.

Another aspect which is not claimed relates to a method of producing a contrast agent in the form of a liquid-dispersible powder comprising nanoscale grains of a matrix material containing a plurality of isolated nanoparticles, wherein at least a portion of the nanoparticles comprise magnetic nanoparticles, wherein each magnetic nanoparticle comprises a magnetic core selected from one of more of Fe or an alloy of Fe/Co encased in a biocompatible FeO shell, the method comprising the steps of:

> a. providing nanoparticles prepared under ultra-high vacuum (UHV) gas phase conditions;
> b. co-depositing the nanoparticles within a matrix under UHV gas phase conditions; and
> c. grinding the film to a fine powder comprising grains of groups of matrix isolated nanoparticles.

Another aspect which is not claimed relates to a magnetic resonance imaging apparatus for diagnosing illness or condition of a patient, the apparatus comprising: a source of a radio frequency pulse operable to expose a patient located within the apparatus to a radio frequency pulse; and a controller operable to vary the field amplitude of the radio frequency pulse.

Suitably, the controller may be operable to vary the field amplitude between a plurality of amplitudes. The controller may be operable to vary the field amplitude of the radio frequency pulse between a first low field amplitude and a second high field amplitude, for example the first low field amplitude may be sufficient to generate an image or series of images of a body part of a user, and the second high field amplitude may be sufficient to cause the magnetic nanoparticles to generate or emit heat.

Another aspect which is not claimed relates to a magnetic resonance imaging apparatus for treating an illness or condition of a patient, the apparatus comprising: a source of a radio frequency pulse operable to expose a patient located within the apparatus to a radio frequency pulse; and a controller operable to vary the field amplitude of the radio frequency pulse.

Suitably, the controller may be operable to vary the field amplitude between a plurality of amplitudes. The controller may be operable to vary the field amplitude of the radio frequency pulse between a first low field amplitude and a second high field amplitude, for example the first low field amplitude may be sufficient to generate an image or series of images of a body part of a user, and the second high field amplitude may be sufficient to cause the magnetic nanoparticles to generate or emit heat.

Another aspect which is not claimed relates to a magnetic resonance imaging apparatus for simultaneously or subsequently diagnosing and treating an illness or condition of a patient to whom the contrast agent or pharmaceutical composition of previous aspects of the invention has been administered, the apparatus comprising: a source of a radio frequency pulse operable to expose a patient located within the apparatus to a radio frequency pulse; and a controller operable to vary the field amplitude of the radio frequency pulse.

Suitably, the controller may be operable to vary the field amplitude between a plurality of amplitudes. The controller may be operable to vary the field amplitude of the radio frequency pulse between a first low field amplitude and a second high field amplitude, for example the first low field amplitude may be sufficient to generate an image or series of images of a body part of a user, and the second high field amplitude may be sufficient to cause the magnetic nanoparticles to generate or emit heat.

Another aspect which is not claimed relates to method of simultaneously or subsequently diagnosing and treating an illness or condition of a patient, the method comprising: administering a contrast agent as defined in any previous aspect of the invention to a patient; positioning the patient within an apparatus as defined in any previous aspect of the invention; operating the controller such that the source of a radio frequency pulse is provided at a first field amplitude sufficient to generate one or more images of one or more parts of the body of the patient; identifying an illness or condition associated with the patient from the generated one or more images; and operating the controller such that the source of a radio frequency pulse is provided at a second field amplitude sufficient to treat the illness or condition associated with the patient. In some embodiments, the step of identifying an illness or condition of the patient may occur prior to the subsequent step of treating the illness or condition.

Another aspect which is not claimed relates to the use of a nanoparticle comprising a core covered at least in part with a layer of metal, in which the core and the layer of metal are comprised or composed of different materials, in the manufacture of a contrast agent.

Another aspect of the invention relates to a nanoparticle comprising a core covered at least in part with a layer of metal, in which the core and the layer of metal are comprised or composed of different materials, for use in diagnosis and/or therapy.

Another aspect of the disclosure, which is not claimed, relates to use of a nanoparticle comprising a core covered at least in part with a layer of metal, in which the core and the layer of metal are comprised or composed of different materials, in diagnosis and/or therapy.

Another aspect of the invention relates to a contrast agent as defined in any previous aspect of the invention, for use in the diagnosis and/or therapy of an illness, condition or disease in a patient. The illness, condition or disease may be selected from one or more of cancer (i.e. tumours), viruses, bacterial infections, inflammatory conditions, or any combination thereof, for example cancer. The cancer may be selected from one or more of carcinoma, sarcoma, melanoma, lymphoma, and leukemia.

Another aspect of the disclosure, which is not claimed, relates to use of a contrast agent as defined in any previous aspect, in the diagnosis and/or therapy of an illness, condition or disease in a patient. The illness, condition or disease may be selected from one or more of cancer (i.e. tumours), viruses, bacterial infections, inflammatory conditions, or any combination thereof, for example cancer. The cancer may be selected from one or more of carcinoma, sarcoma, melanoma, lymphoma, and leukemia.

Another aspect which is not claimed relates to a method of diagnosing and/or treating an illness or condition in a patient comprising administration of a nanoparticle comprising a core covered at least in part with a layer of metal, in which the core and the layer of metal are comprised or composed of different materials.

Another aspect which is not claimed relates to a method of diagnosing and/or treating an illness or condition in a patient comprising administration of a contrast agent as defined in any previous aspect of the invention.

Another aspect which is not claimed relates to a nanoparticle comprising a core covered at least in part with a layer of metal, in which the core and the layer of metal are comprised or composed of different materials, for use as a medicament.

Another aspect of the invention relates to a contrast agent as defined in any previous aspect of the invention, for use as a medicament.

Another aspect which is not claimed relates to nanoparticles comprising a core covered at least in part with a layer of metal, in which the core and the layer of metal are comprised or composed of different materials for use in the treatment of cancer.

Another aspect of the invention relates to a contrast agent according to claim 1 comprising a plurality of magnetic nanoparticles, wherein each magnetic nanoparticle comprises a core covered at least in part with a layer of metal, wherein the core and the layer of metal are comprised or composed of different materials; and one or more pharmaceutically acceptable carriers, for use in the treatment of cancer.

Throughout the description and claims of this specification, the words "comprise" and "contain" and variations of the words, for example "comprising" and "comprises", mean "including but not limited to", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

Brief Description of Drawings

The present invention will now be described by way of example only with reference to the following Figures, of which:

Figure 1A illustrates the formation of a known magnetic structure;
Figure 1B contains a graph of magnetic moment per atom as a function of the Fe volume fraction for magnetic structures having Fe nanoparticles in a Co matrix and Co nanoparticles in an Fe matrix;
Figure 2A shows in block diagram form apparatus for forming a magnetic structure according to the present invention;

Figure 2B shows apparatus for coating a core of a nanoparticle;

Figure 3 illustrates the method of simultaneously or subsequently diagnosing and treating an illness or condition of a patient in a single operation of an apparatus according to one embodiment of the present invention;

Figure 4 illustrates the relationship between the power dissipated in the tissue by a radio frequency pulse having a field frequency f and amplitude Ho, conductivity of tissue (Sm-1) $\sigma$, and magnetic susceptibility of the tissue

Description of Embodiments

Apparatus for and a process of forming a known magnetic structure comprising a matrix with embedded particles formed from a ferromagnetic transition metal have been described above with reference to Figures 1A and 1B.

According to one embodiment of the present invention, the contrast agent of the present invention comprises a plurality of magnetic nanoparticles. Each magnetic nanoparticle comprises a core covered at least in part by a layer of metal. The core is composed of ferromagnetic material. The layer of metal and the core are composed of different materials. According to a further embodiment, the contrast agent comprises a plurality of magnetic nanoparticles. Each magnetic nanoparticle comprises a core covered at least in part with a layer of metal; and a matrix. The magnetic nanoparticles are embedded in the matrix. The matrix and the core is composed of ferromagnetic material. The core and the layer of metal are composed of different materials.

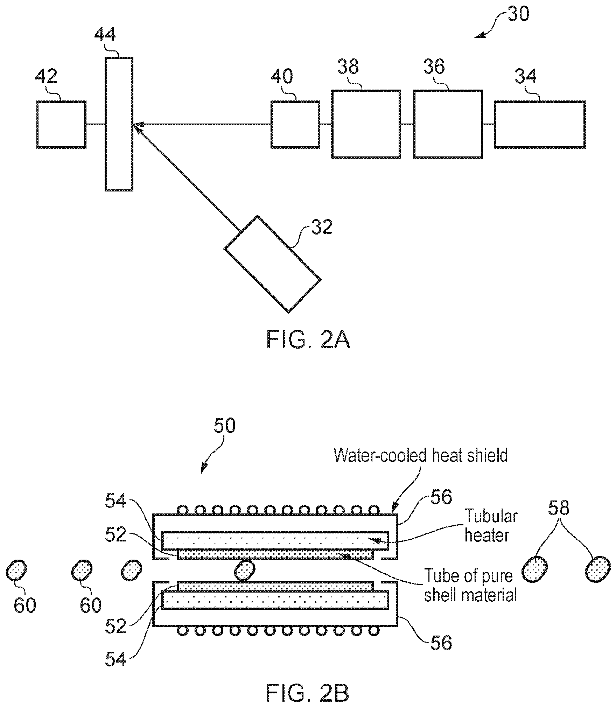

Figure 2A shows in block diagram form apparatus 30 for producing the contrast agent of the present invention. The apparatus 30 comprises an MBE source 32, a thermal gas aggregation source 34, a first thermal evaporator 36, a second thermal evaporator 38 and a venturi 40. The apparatus 30 further comprises refrigeration apparatus 42 which is operative with liquid nitrogen in certain examples to refrigerate a substrate 44 and its environs. The thermal gas aggregation source 34 and the first and second thermal evaporators 36, 38 are operative in the same vacuum. As is described further below the MBE source 32 is operative to generate an atomic beam of matrix material and the thermal gas aggregation source 34 is operative at the same time to generate a beam of nanoparticles. The two beams are deposited simultaneously on the substrate 44 to form a magnetic structure in the form of a thin film matrix formed from deposited matrix material with nanoparticles distributed through and embedded in the matrix. The substrate 44 constitutes a component forming part or to form part of a product. According to one application example the substrate 44 is constituted by part of a roll of material in reel to reel coating apparatus.

According to another application example the substrate 44 forms part or will form part of the like of an electric motor or mobile telephone. According to a further application example the substrate 44 is constituted by one of several strategic locations on a critical magnetic component in electro-mechanical apparatus or the like. In use the magnetic structure is operative to amplify the magnetic field of the magnetic component.

The first and second thermal evaporators 36, 38 of Figure 2A are of the same form and function. Figure 2B provides a detailed view of the first and second thermal evaporators 50. The thermal evaporator 50 is of generally tubular form such that it defines a bore through which a beam of nanoparticles may pass. The thermal evaporator 50 comprises a tube of pure material 52 which is to be deposited as a layer on each of the nanoparticles passing through the thermal evaporator. The thermal evaporator 50 further comprises a tubular heater 54 which surrounds and is adjacent the tube of pure material 52. A water cooled heat shield 56 surrounds the outwardly directed surface of the tubular heater 54 and the end faces of the tubular heater 54 and the tube of pure material 52. In use the thermal evaporator 50 is operative to vaporise the pure material 52 with the material vapour being present in the bore of the thermal evaporator. A beam of uncoated nanoparticles 58 is received at one end of the bore of the thermal evaporator 50 and on passing through the material vapour in the bore the nanoparticles are coated with a layer of the material. The coated nanoparticles 60 then leave the other end of the bore of the thermal evaporator. In forms of the apparatus 30 nanoparticles are coated with only one layer of material. According to such forms the second thermal evaporator 38 of the apparatus of Figure 2A is either absent or inoperative. In other forms of the apparatus 30 nanoparticles are coated with first and second layers of the same or different material. According to such forms the first thermal evaporator 36, 50 comprises a tube of a first material 52 and the second thermal evaporator 38, 50 comprises a tube of the first material or a second different material 52. In further forms of the apparatus 30 nanoparticles are coated with third and further layers of the same or different material. According to such further forms the apparatus 30 comprises thermal evaporators which correspond in number to the number of layers to be deposited on the nanoparticles with the plural thermal evaporators disposed in line such that the beam of nanoparticles can pass in turn through the bore of each of the thermal evaporators. A first example of a process of forming a magnetic structure on the substrate will now be described with reference to Figures 2A and 2B.

According to the first example only one layer of material is deposited on the nanoparticles. As stated above the second thermal evaporator 38 of Figure 2A is therefore either absent or inoperative. The thermal gas aggregation source 34 is operative to generate a beam of Fe nanoparticles of diameters in the range of 1 nm to 5 nm. The diameter of the Fe nanoparticles is determined by controlling the power level and the gas pressure of the thermal gas aggregation source 34. The beam of Fe nanoparticles passes through the bore of the first thermal evaporator 36 which comprises a tube 52 of either Co or Ag. Each Fe nanoparticle is therefore coated with a layer of either Co or Ag to a thickness of between 1 and 10 atomic layers. The operative temperature of the first thermal evaporator 36 is determined by the material to be deposited. The operative temperature for Ag is about 800 °C. As mentioned above the thickness of the layer depends on the velocity of the nanoparticles, which cannot be controlled, and the temperature. If it is desired to increase the thickness of the layer the operative temperature need only be increased slight because the vapour pressure is very sensitive to temperature. For example, to double the thickness of an Ag layer it is only necessary to increase the temperature by about 50 °C. In such composite nanoparticles Fe constitutes the core of the nanoparticles. The MBE source 32 is operative at the same time as the thermal gas aggregation source 34 to generate an atomic beam of either Co or Ag such that the atomic beam is of the same material as the coating on the Fe nanoparticles. The atomic beam and the beam of nanoparticles are deposited simultaneously on the substrate 44 to form a magnetic structure comprising a matrix formed by the atomic beam in which nanoparticles are embedded. The layer of material on the Fe core decreases the likelihood of the Fe cores coming into contact with one another. By way of example and to provide a comparison with the performance of uncoated cores as described above with reference to Figure 1 B, if the Fe cores have a diameter of 5nm and the coating is of a single atomic layer of 0.2 nm the volume fraction of the core can be increased to 66% without agglomeration compared to about 20% if uncoated Fe nanoparticles are used. A second example of process of forming a magnetic structure on the substrate will now be described with reference to Figures 2A and 2B.

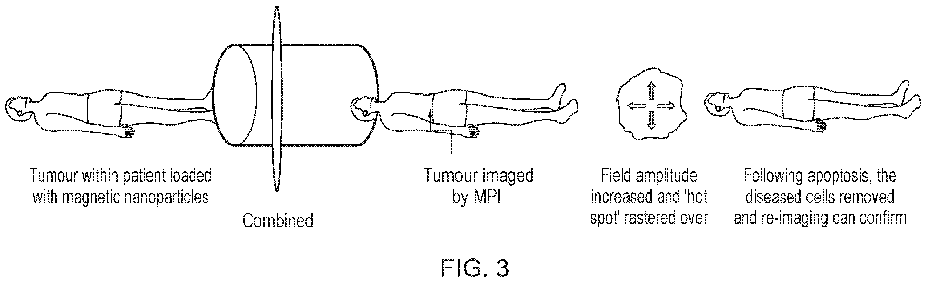

According to the second example two layers of material are deposited in turn on the nanoparticles. As stated above the second thermal evaporator 38 of Figure 2A is therefore operative. The thermal gas aggregation source 34 is operative to generate a beam of Co nanoparticles of diameters in the range of 1 nm to 5 nm. The diameter of the Co nanoparticles is determined by controlling the power level and the gas pressure of the thermal gas aggregation source 34. The beam of Co nanoparticles passes through the bore of the first thermal evaporator 36 which comprises a tube 52 of an anti-ferromagnetic material such as Cr or Mn. Each Co nanoparticle is therefore coated with a layer of either Cr or Mn to a thickness of between 1 and 10 atomic layers. Then the nanoparticles pass through the bore of the second thermal evaporator 38 which in one form comprises a tube 52 of a rare earth metal such as Ho or Dy. Each nanoparticle is therefore coated with a second layer of either Ho or Dy to a thickness of between 1 and 10 atomic layers. In another form the nanoparticles pass through the bore of the second thermal evaporator 38 which comprises a tube 52 of the same anti-ferromagnetic material as the first thermal evaporator 36. The operative temperatures of the first and second thermal evaporators 36, 38 are determined by the material to be deposited. Figure 3 shows a perspective view of a Co core coated with a layer of each of Cr and a rare earth metal (i.e. Ho or Dy). Figure 3 shows a section through a coated nanoparticle 70 with Co forming the core 72, Cr forming a layer immediately over the Co core and either Ho or Dy forming an exterior layer immediately over the Cr layer. Figure 3 further shows a beam of nanoparticles 78 after deposition of the Cr layer and Ho or Dy layer. The MBE source 32 is operative at the same time as the thermal gas aggregation source 34 to generate an atomic beam of either Ho or Dy such that the atomic beam is of the same material as the outer coating on the Co nanoparticles. The atomic beam and the beam of nanoparticles are deposited simultaneously on the substrate 44 to form a magnetic structure comprising a matrix formed by the atomic beam in which nanoparticles are embedded.

A third example of process of forming a magnetic structure on the substrate will now be described with reference to Figures 2A and 2B. According to the third example only one layer of material is deposited on the nanoparticles. As stated above the second thermal evaporator 38 of Figure 2A is therefore either absent or inoperative. The thermal gas aggregation source 34 is operative to generate a beam of Fe nanoparticles of diameters in the range of 1 nm to 5 nm. The beam of Fe nanoparticles passes through the bore of the first thermal evaporator 36 which comprises a tube 52 of either Au or Ag. Each Fe nanoparticle is therefore coated with a layer of either Au or Ag to a thickness of between 1 and 10 atomic layers. The operative temperature of the first thermal evaporator 36 is determined by the material to be deposited. A thermal evaporator is employed in the apparatus of Figure 2 instead of the MBE source 32. The thermal evaporator is operative on a body of water to direct water vapour such that it impinges upon the substrate 44. The substrate is refrigerated by the refrigeration apparatus 42 whereby the impinging water vapour is deposited as ice on the substrate 44. The ice and the nanoparticles are deposited simultaneously on the substrate 44 to form a magnetic structure comprising an ice matrix in which nanoparticles are embedded. When the magnetic structure is formed the temperature is raised to room temperature to provide a liquid containing the nanoparticles. The liquid is then sprayed onto a desired surface to deposit the nanoparticles upon the surface.

With reference to the Figure 3, the apparatus is a magnetic resonance imaging machine 1 comprising a central cavity dimensioned to receive at least one body part, and in some instances the entire body of a patient. The magnetic resonance imaging machine 1 comprises a source of a radio frequency pulse (not shown) operable to expose a patient located within the central cavity of the apparatus to a radio frequency pulse. The magnetic resonance imaging machine 1 comprises a controller (not shown) operable to vary the field amplitude of the radio frequency pulse.

A contrast agent of the present invention as described herein is administered, preferably intravenously, to the patient. It is however to be understood that the contrast agent may be administered by any suitable means. Furthermore, the contrast agent may become concentrated within the region of affected tissue by direct injection in the localised region, due to enhanced permeability retention effect of the affect tissue, and/or by application of an external magnetic field.

The controller is set to produce a first low field amplitude radio frequency pulse having a first low field frequency. The radio frequency pulse (low field amplitude and low frequency) causes the magnetic nanoparticles of the contrast agent to alter their magnetization alignment relative to the field. In response to the force bringing them back to their equilibrium orientation, the magnetic nanoparticles undergo a rotating motion (precession). These changes in magnetization alignment cause a changing magnetic flux, which yields a changing voltage in receiver coils to give the signal. The frequency at which the magnetic nanoparticles of the contrast agent resonate depends on the strength of the local magnetic field around the magnetic nanoparticles. By applying additional magnetic fields (gradients) that vary linearly over space, specific slices to be imaged can be selected, and an image is obtained by taking the 2-D Fourier transform of the spatial frequencies of the signal (a.k.a., k-space).

Application of the radio frequency pulse at the first low field amplitude and frequency generates a first image or series of images of a desired body portion or entire body of the patient. The location of diseased tissue, such as tumours, can be detected because the magnetic nanoparticles within the contrast agent adj acent or in different tissues return to their equilibrium state at different rates (i.e., they have different relaxation times). By changing the parameters on the scanner this effect is used to create contrast between different types of body tissue.

An illness or condition of the patient may therefore be diagnosed during application of the radiofrequency pulse at the first low field amplitude. Once the illness or condition has been diagnosed and accurately located, the controller is operated to generate a radio frequency pulse at a second high field amplitude and second high field frequency directed selectively towards the target area or areas of the body for treatment.

The second high field amplitude radio frequency pulse causes the magnetic nanoparticles to generate a hot spot of localised heat within the targeted region of the body. The radio frequency pulse is rastered, moved back and forth repeatedly across the target region, creating a localised heat spot which also moves back and forth repeatedly across the target region. The raster effect causes a selective localised heat treatment in the target area of illness, condition or disease. The apparatus of the present invention is therefore able to selectively target diseased body areas or tissue of a patient enabling selective treatment.

The apparatus of the present invention enables the identification of diseased tissue within a body of a patient followed by subsequent selective and localised treatment of the diseased tissue during a single operation of the apparatus.

Once the contrast agent has emitted heat selectively at the desired location of a user's body, the damaged tissue undergoes apoptosis with reduced damage to surrounding healthy tissue. Once the treatment has been completed, the controller may be further operated to once again generate a radio frequency pulse at the first low field amplitude and first frequency to generate a further series of images of the diseased body part or tissue to determine whether the treatment has been successful or whether further rounds of exposure to a radio frequency pulse at a second high field amplitude are required. The further images are assessed and if necessary further treatment is provided by once again operating the controller to generate a radio frequency pulse at the second high field amplitude and second frequency.

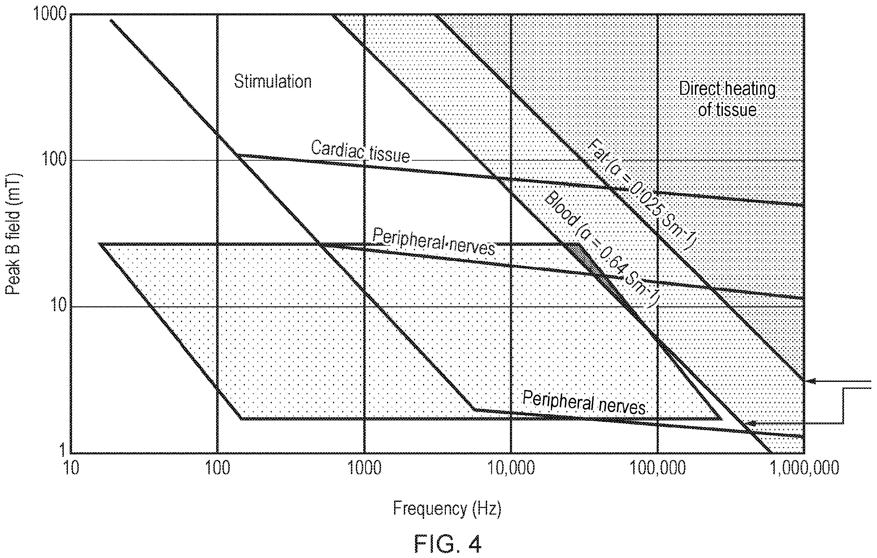

Figure 4 demonstrates the relationship between the power dissipated by the heat spot in the localised tissue by the radio frequency pulse having a frequency f and amplitude $H_0$.

$$\nabla \times E = -\partial B / \partial t$$

$$P = \pi^2 (\mu^2 \mu_0^2 \sigma / 2) f^2 H_0^2 r^2$$

It can be seen from the equation and the Figure that the critical parameter is the product of the frequency (f) and the amplitude (Ho) of the pulse. The safe zone for providing direct heating to tissue is indicated in the Figure.

Disclosed but not claimed is an apparatus and a method for simultaneously or subsequently diagnosing and treating an illness or condition of a patient during a single operation of the apparatus. Disclosed but not claimed is an apparatus and a method for accurately identifying, diagnosing and locating an illness or condition of a patient while simultaneously or subsequently accurately locating and treating the illness or condition of a patient. Disclosed but not claimed is an apparatus and a method with improved selectivity for treating an illness or condition of a patient. The apparatus and method therefore enables an illness or condition of a patient to be identified and treated, either simultaneously or subsequently, during a single operation of the apparatus and as such reduces the number of hospital appointments required by the patient and reduces the attendance time of a medical practitioner. As a result of the improved selectivity of the apparatus and method for treating an illness or condition of a patient, the recovery time of a patient is significantly reduced.

## Claims

1.  A contrast agent for magnetic resonance imaging, in which the contrast agent comprises:

    a matrix
    a plurality of magnetic nanoparticles embedded in the matrix, wherein each magnetic nanoparticle comprises a core covered at least in part with a layer of metal, wherein the core and the layer of metal are comprised of different materials, wherein the matrix and the core comprise a ferromagnetic material, wherein the core comprises a transition metal, wherein the layer of metal comprises an antiferromagnetic material, and wherein the matrix material comprises a rare earth metal; and
    one or more pharmaceutically acceptable carriers.

2.  The contrast agent of claim 1, wherein the rare earth metal is selected from Dy or Ho.

3.  The contrast agent of claim 1, wherein the layer of metal comprises an antiferromagnetic transition metal.

4.  The contrast agent of claim 3, wherein the ferromagnetic transition metal is selected from Fe, Co, or Ni.

5.  The contrast agent of claim 3, wherein the antiferromagnetic transition metal is selected from Cr or Mn.

6.  The contrast agent of any one of claims 1 to 5, comprising a second layer which covers the layer of metal at least in part, the second layer being formed from a rare earth metal, optionally wherein the second layer and the matrix comprise the same rare earth metal.

7.  The contrast agent of any preceding claim, wherein each magnetic nanoparticle is of a diameter of no more than one of 10 nm, 5 nm, 4 nm, 3 nm, 2 nm, or 1 nm.

8.  The contrast agent of any preceding claim, wherein each magnetic nanoparticle is of a diameter between 1 nm and 5 nm.

9.  A process for producing a contrast agent for use in magnetic resonance imaging, the process comprising:

    forming magnetic nanoparticles by depositing at least in part a layer of metal on a core, wherein the core comprises a ferromagnetic transition metal, and the layer of metal comprises an antiferromagnetic material;
depositing a matrix material onto a substrate to thereby form a matrix, wherein the matrix is formed from a ferromagnetic rare earth metal;
depositing the magnetic nanoparticles onto the matrix as the matrix forms to thereby embed the magnetic nanoparticles in the matrix; and
combining with one or more pharmaceutically acceptable carriers.

10. A contrast agent according to any one of claims 1 to 8, for use in diagnosis or therapy.

11. A contrast agent according to claim 10, for use in the diagnosis or treatment of cancer, viruses, bacterial infections, inflammatory conditions, or any combination thereof, optionally wherein the cancer is selected from one or more of carcinoma, sarcoma, melanoma, lymphoma, and leukemia.

## Patentansprüche

1.  Kontrastmittel für Magnetresonanzbildgebung, wobei das Kontrastmittel Folgendes umfasst:

    eine Matrix
    eine Mehrzahl von in der Matrix eingebetteten magnetischen Nanopartikeln, wobei jedes magnetische Nanopartikel einen Kern umfasst, der wenigstens teilweise mit einer Metallschicht bedeckt ist, wobei der Kern und die Metallschicht aus unterschiedlichen Materialien bestehen, wobei die Matrix und der Kern ein ferromagnetisches Material umfassen, wobei der Kern ein Übergangsmetall umfasst, wobei die Metallschicht ein antiferromagnetisches Material umfasst und wobei das Matrixmaterial ein Seltenerdmetall umfasst; und
    einen oder mehrere pharmazeutisch akzeptable Träger.

2.  Kontrastmittel nach Anspruch 1, wobei das Seltenerdmetall aus Dy und Ho ausgewählt ist.

3.  Kontrastmittel nach Anspruch 1, wobei die Metallschicht ein antiferromagnetisches Übergangsmetall umfasst.

4.  Kontrastmittel nach Anspruch 3, wobei das ferromagnetische Übergangsmetall aus Fe, Co und Ni ausgewählt ist.

5.  Kontrastmittel nach Anspruch 3, wobei das antiferromagnetische Übergangsmetall aus Cr und Mn ausgewählt ist.

**6.** Kontrastmittel nach einem der Ansprüche 1 bis 5, das eine zweite Schicht umfasst, die die Metallschicht wenigstens teilweise bedeckt, wobei die zweite Schicht aus einem Seltenerdmetall gebildet ist, wobei die zweite Schicht und die Matrix optional das gleiche Seltenerdmetall umfassen.

**7.** Kontrastmittel nach einem vorherigen Anspruch, wobei jedes magnetische Nanopartikel einen Durchmesser von nicht mehr als 10 nm, 5 nm, 4 nm, 3 nm, 2 nm oder 1 nm hat.

**8.** Kontrastmittel nach einem vorherigen Anspruch, wobei jedes magnetische Nanopartikel einen Durchmesser zwischen 1 nm und 5 nm hat.

**9.** Verfahren zur Herstellung eines Kontrastmittels zur Verwendung in Magnetresonanzbildgebung, wobei das Verfahren Folgendes beinhaltet:

Bilden von magnetischen Nanopartikeln durch wenigstens teilweises Abscheiden einer Metallschicht auf einen Kern, wobei der Kern ein ferromagnetisches Übergangsmetall umfasst und die Metallschicht ein antiferromagnetisches Material umfasst;
Abscheiden eines Matrixmaterials auf ein Substrat, um dadurch eine Matrix zu bilden, wobei die Matrix aus einem ferromagnetischen Seltenerdmetall gebildet wird;
Abscheiden der magnetischen Nanopartikel auf die Matrix, wenn sich die Matrix bildet, um dadurch die magnetischen Nanopartikel in die Matrix einzubetten; und
Kombinieren mit einem oder mehreren pharmazeutisch akzeptablen Trägern.

**10.** Kontrastmittel nach einem der Ansprüche 1 bis 8 zur Verwendung in der Diagnose oder Therapie.

**11.** Kontrastmittel nach Anspruch 10 zur Verwendung in der Diagnose oder Behandlung von Krebs, Viren, bakteriellen Infektionen, entzündlichen Zuständen oder einer beliebigen Kombination davon, wobei der Krebs optional aus einem oder mehreren aus Karzinom, Sarkom, Melanom, Lymphom und Leukämie ausgewählt ist.

**Revendications**

**1.** Agent de contraste pour imagerie par résonance magnétique, l'agent de contraste comprenant:

une matrice ;
une pluralité de nanoparticules magnétiques intégrées dans la matrice, chaque nanoparticule magnétique comprenant un noyau recouvert, au moins partie, d'une couche de métal, le noyau et la couche de métal étant constitués de différents matériaux, la matrice et le noyau comprenant un matériau ferromagnétique, le noyau comprenant un métal de transition, la couche de métal comprenant un matériau anti-ferromagnétique et le matériau de la matrice comprenant un métal terreux rare ; et
un ou plusieurs supports pharmaceutiquement acceptables.

**2.** Agent de contraste selon la revendication 1, dans lequel le métal terreux rare est sélectionné parmi le Dy et le Ho.

**3.** Agent de contraste selon la revendication 1, dans lequel la couche de métal comprend un métal de transition anti-ferromagnétique.

**4.** Agent de contraste selon la revendication 3, dans lequel le métal de transition anti-ferromagnétique est choisi parmi le Fe, le Co et le Ni.

**5.** Agent de contraste selon la revendication 3, dans lequel le métal de transition anti-ferromagnétique est choisi parmi le Cr ou le Mn.

**6.** Agent de contraste selon l'une quelconque des revendications 1 à 5, comprenant une seconde couche qui recouvre la couche de métal au moins en partie, la seconde couche étant formée d'un métal terreux rare, la seconde couche et la matrice comprenant éventuellement le même métal terreux rare.

**7.** Agent de contraste selon l'une quelconque des revendications précédentes, dans lequel chaque nanoparticule magnétique a un diamètre qui n'est pas supérieur à 10 nm, 5 nm, 4 nm, 3 nm, 2 nm ou 1 nm.

**8.** Agent de contraste selon l'une quelconque des revendications précédentes, dans lequel chaque nanoparticule magnétique a un diamètre compris entre 1 et 5 nm.

**9.** Procédé de production d'un agent de contraste destiné à être utilisé dans une imagerie par résonance magnétique, le procédé comprenant les étapes consistant à :

former des nanoparticules magnétiques en déposant, au moins en partie, une couche de métal sur un noyau, le noyau comprenant un métal de transition ferromagnétique et la couche de métal comprenant un matériau anti-ferromagnétique ;
déposer un matériau matriciel sur un substrat pour former ainsi une matrice, la matrice étant formée d'un métal terreux rare ferromagnétique ;

déposer les nanoparticules magnétiques sur la matrice au fur et à mesure de la formation de la matrice pour intégrer ainsi les nanoparticules magnétiques dans la matrice ; et

combiner avec un ou plusieurs supports pharmaceutiquement acceptables.

10. Agent de contraste selon l'une quelconque des revendications 1 à 8, destiné à être utilisé dans un diagnostic ou une thérapie.

11. Agent de contraste selon la revendication 10, destiné à être utilisé dans le diagnostic ou le traitement d'un cancer, de virus, d'infections bactériennes, d'états inflammatoires ou d'une quelle combinaison correspondante, le cancer étant éventuellement choisi parmi un carcinome et/ou un sarcome et/ou un mélanome et/ou un lymphome et/ou une leucémie.

Cluster source

MBE source

14

16

18

20

12    10

FIG. 1A

Co    Fe

Fe    Co

Fe$_{25}$Co$_{75}$

Slater-
Pauling
curve

Magnetic moment atom ($\mu_B$)

3.0

2.5

2.0

1.5

1.0

0    20    40    60    80    100

Fe volume fraction (%)

FIG. 1B

FIG. 2A

Water-cooled heat shield

Tubular heater

Tube of pure shell material

FIG. 2B

Tumour within patient loaded with magnetic nanoparticles

Combined

Tumour imaged by MPI

Field amplitude increased and 'hot spot' rastered over

Following apoptosis, the diseased cells removed and re-imaging can confirm

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008057001 A1
- US 2012168669 A1
- US 2016271274 A1
- GB 2543604 A